(19) Europäisches Patentamt
European Patent Office
Office européen des brevets

(11) EP 4 785 964 A1

(12) **EUROPEAN PATENT APPLICATION**
published in accordance with Art. 153(4) EPC

(43) Date of publication:
**05.08.2026 Bulletin 2026/32**

(21) Application number: **24873050.9**

(22) Date of filing: **27.09.2024**

(51) International Patent Classification (IPC):
***A61K 48/00*** (2006.01) ***A61K 38/17*** (2006.01)
***A61P 35/00*** (2006.01) ***C12N 15/67*** (2006.01)
***C07K 14/47*** (2006.01) ***C12N 15/11*** (2006.01)
***C12N 15/113*** (2010.01) ***C12N 15/63*** (2006.01)

(52) Cooperative Patent Classification (CPC):
**A61K 38/17; A61K 48/00; A61P 35/00; C07K 14/47; C12N 15/11; C12N 15/113; C12N 15/63; C12N 15/67**

(86) International application number:
**PCT/KR2024/014752**

(87) International publication number:
**WO 2025/071343 (03.04.2025 Gazette 2025/14)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **27.09.2023 KR 20230130728**
**29.03.2024 KR 20240043273**

(71) Applicant: **Hanmi Pharm. Co., Ltd.**
**Hwaseong-si, Gyeonggi-do 18536 (KR)**

(72) Inventors:
- **KIM, Inn Ah**
  **Hwaseong-si, Gyeonggi-do 18469 (KR)**
- **BYUN, Joo Yun**
  **Hwaseong-si, Gyeonggi-do 18469 (KR)**
- **SHIN, Seung Hyun**
  **Hwaseong-si, Gyeonggi-do 18469 (KR)**
- **LEE, Ji Hee**
  **Hwaseong-si, Gyeonggi-do 18469 (KR)**
- **LIM, Chang Gyu**
  **Hwaseong-si, Gyeonggi-do 18469 (KR)**
- **HAN, Young Jin**
  **Hwaseong-si, Gyeonggi-do 18469 (KR)**
- **HEO, Yong Ho**
  **Hwaseong-si, Gyeonggi-do 18469 (KR)**

(74) Representative: **Santarelli**
**Tour Trinity**
**1 bis Place de la Défense**
**92400 Courbevoie (FR)**

Remarks:
The complete document including Reference Table(s) and the Sequence Listing(s) can be downloaded from the EPO website

# (54) PHARMACEUTICAL COMPOSITION FOR PREVENTING OR TREATING CANCER COMPRISING NUCLEIC ACID STRUCTURE ENCODING P53 PROTEIN

(57) Provided are a messenger RNA (mRNA) including a 5' untranslated region (5'UTR), a sequence encoding a p53 protein, and a 3' untranslated region (3'UTR), a composition for delivering the mRNA or p53 to a subject, comprising the mRNA, and a composition and a method for preventing or treating cancer.

EP 4 785 964 A1

# FIG. 1

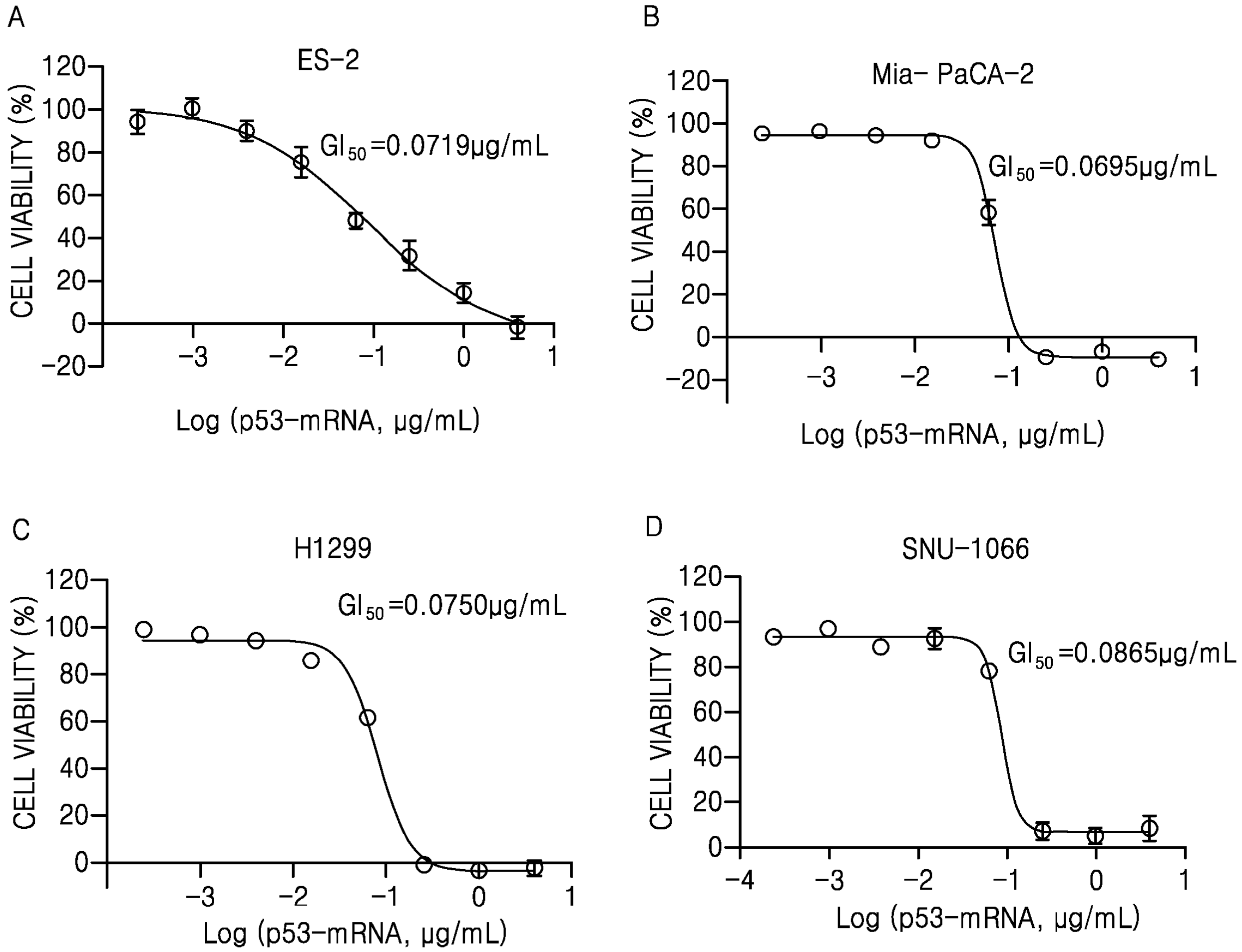
A
ES-2
GI50=0.0719μg/mL
CELL VIABILITY (%)
Log (p53-mRNA, μg/mL)
B
Mia- PaCA-2
GI50=0.0695μg/mL
CELL VIABILITY (%)
Log (p53-mRNA, μg/mL)
C
H1299
GI50=0.0750μg/mL
CELL VIABILITY (%)
Log (p53-mRNA, μg/mL)
D
SNU-1066
GI50=0.0865μg/mL
CELL VIABILITY (%)
Log (p53-mRNA, μg/mL)

## Description

### Technical Field

The present disclosure relates to a nucleic acid construct encoding a p53 protein and use thereof.

### Background Art

RNA such as messenger RNA is frequently used as a gene delivery molecule in the field of cancer immunotherapy and stem cell-based biomedical research, as an alternative to plasmid DNA. As a direct source of gene products, mRNA has several advantages including the lack of a requirement for nuclear entry, which poses a significant barrier to DNA delivery, especially in non-dividing cells. Also, by avoiding the expression of oncogenes caused by abnormal transcription and insertional mutagenesis, mRNA is less likely to integrate into the host genome.

For a given gene, untranslated regions (UTRs) of the gene including a 5'UTR and a 3'UTR are regions involved in regulating expression. The 5'UTR is a DNA regulatory region at the 5' end of a protein-coding sequence, and is transcribed into mRNA but is not translated into a protein. The 5'UTR may include various regulatory elements, such as a 5'-cap structure, a G-quadruplex structure (G4), a stem-loop structure, and an internal ribosome entry site (IRES), and such regulatory elements play an important role in controlling translation initiation. The 3'UTR positioned downstream of the protein-coding sequence is known to be involved in many regulatory processes including transcript cleavage, stability and polyadenylation, translation, and mRNA localization. The 3'UTR serves as a binding site for many regulatory proteins and small non-coding RNAs (e.g., microRNAs).

p53 is a tumor suppressor protein that in humans is encoded by the TP53 gene. p53, as a tumor suppressor, is important for cancer prevention in the cell cycle of multicellular organisms.

Current mRNA therapies are generally not tissue-specific or rely on native or standard UTR sequences that provide a low level of protein expression. In this regard, there is a need for novel UTRs and a combination thereof that can stabilize mRNA therapies and increase synthesis of the p53 protein.

### Disclosure of Invention

### Technical Problem

An aspect provides isolated mRNA including a nucleotide sequence encoding a non-natural 5' untranslated region (5'UTR), a nucleotide sequence encoding p53 or a functional fragment thereof, and a nucleotide sequence encoding a 3'UTR, each of which is operatively linked to one another.

Another aspect provides a method of obtaining p53 or a functional fragment thereof, the method including translating the mRNA.

Another aspect provides a composition including the mRNA as an active ingredient.

Another aspect provides a composition for preventing or treating cancer in a subject, the composition including the mRNA as an active ingredient.

Another aspect provides a method of utilizing the mRNA, the method including introducing the mRNA to a host cell.

Another aspect provides DNA encoding the mRNA.

Another aspect provides a method of obtaining RNA, the method including transcribing RNA by using the DNA as a template.

### Solution to Problem

As used in the present specification, "5' untranslated region (5'-UTR)" refers to an mRNA region where a polypeptide is not encoded, directly upstream (i.e., 5') of the first codon, i.e., an initiation codon, of an mRNA transcript to be translated by ribosomes.

As used in the present specification, "3' untranslated region (3'-UTR)" refers to an mRNA region where a polypeptide is not encoded, directly downstream (i.e., 3') of a stop codon, i.e., a termination codon, of an mRNA transcript signaling termination of translation.

As used in the present specification, "open reading frame (ORF)" or "sequence encoding a polypeptide" refers to a continuous region of DNA that starts with an initiation codon, such as a methionine codon (ATG) and ends with a stop codon, such as TAA, TAG, or TGA, and encodes a polypeptide.

As used in the present specification, "polyadenylate sequence," "poly (A)," or "poly (A) tail" refers to an mRNA region including multiple continuous adenine nucleotides, downstream of the 3'-UTR, for example, directly downstream

(i.e., 3') of the 3'-UTR. The poly (A) tail may include 1,000, 500, 400, or 300 or less adenine nucleotides. For example, the poly (A) tail may include 10 to 300 adenine nucleotides. For example, the poly (A) tail may include 10, 20, 30, 40, 50, 60, 70, 80, 90, 100, 110, 120, 130, 140, 150, 160, 170, 190, 200, 210, 220, 230, 240, 250, 260, 270, 280, 290, or 300 nucleotides. The poly (A) tail may include, for example, 50 to 250 adenine nucleotides. The poly (A) tail in a living body, such as a cell or a subject, may serve to protect the mRNA from an attack of an enzyme, such as a cytoplasmic enzyme, and aid in termination of transcription, export of the mRNA from the nucleus, and translation. A 3' poly (A) tail refers to a region (stretch) of the adenine nucleotides, and is generally added to mRNA that is transcribed during mRNA processing or to immature mRNA.

As used in the present specification, the term "operatively linked" refers to connection of nucleotide sequences on a single nucleic acid fragment so that a function is affected by another. For example, a promoter is operatively linked to a coding sequence (e.g., an ORF) when the promoter is capable of affecting the expression of the coding sequence (that is, when transcription of the coding sequence is regulated by the promoter). The coding sequence may be operatively linked to a regulatory sequence in a sense direction or an anti-sense direction. Unless otherwise stated in the present specification, each nucleic acid sequence included in the claimed mRNA or DNA is operatively linked to one another.

As used in the present specification, unless a location of a nucleotide sequence is otherwise stated, a nucleotide sequence is connected or located from the 5'-end to the 3'-end.

As used in the present specification, the term "vector" or "nucleic acid construct" refers to any nucleic acid capable of carrying a gene, an ORF, or a DNA fragment into a cell. The vector may be, for example, one which may be replicated in a cell. The vector may be a virus, a bacteriophage, a provirus, a plasmid, a phagemid, a transposon, or an artificial chromosome such as an yeast artificial chromosome (YAC), a bacterial artificial chromosome (BAC), a plant artificial chromosome (PLAC), etc.

The first aspect provides mRNA including a non-natural 5'UTR, a sequence encoding a polypeptide, and a non-natural 3'UTR,

wherein the 5'UTR does not form a secondary structure,
the sequence encoding a polypeptide is a sequence encoding p53 or a functional fragment thereof, and
the 3'UTR has: a secondary structure represented by dot-bracket notation below: .(((((..........(((...(((((.(((....))).)))))...)))..(((((((..((((....((((((.(((((.......)))))..))).)))))))...))))-)))......(((....)))...........(((.........(((....))))))..))))); or
a secondary structure represented by dot-bracket notation below: (((((((((((...(((((.((........((((((((.........))))))))))))))).....(((...))).....))))))))))))..((((((((((((....) )))))...)))))).....((((((...)))))).........
wherein, in the dot-bracket notation, a dot denotes unpaired bases in a sequence, and "(" and ")" denote paired bases in a sequence. As used in the present specification, the term "secondary structure" refers to binding between paired bases.

The mRNA including the non-natural 5'UTR may have a sequence of Formula 1:

Formula 1: $AGN_aGCCACC$

wherein N is A, U, G, or C, each N is identical to or different from each other, and a indicates the number of repeats of N and is 42 or 62.

The mRNA including the 3'UTR may have a mean free energy of -0.1 to -0.3 kcal/mol for RNA folding,

(a) the secondary structure of the mRNA comprising the 3'UTR, represented by .(((((..........(((...(((((.(((....))).)))))...)))..(((((((..((((....((((((.(((((.......)))))..))).)))))))...))))-)))......(((....)))...........(((.........(((....))))))..))))) has a length of 178 nt and a GC-content (GC%) of 40 to 50 %, and
(b) the secondary structure of the mRNA comprising the 3'UTR, represented by (((((((((((...(((((.((........((((((((.........))))))))))))))).....(((...))).....))))))))))))..((((((((((((....) )))))...)))))).....((((((...)))))).......... has a length of 158 nt and a GC% of 30 to 50 %.

The 5'UTR may include a nucleotide sequence of any one of SEQ ID NOs: 1 to 9.

Also, the 3'UTR may include a nucleotide sequence of any one of SEQ ID NOs: 10 to 17. The 3'UTR having the secondary structure of (a) may include a nucleotide sequence of any one of SEQ ID NOs: 10 to 14. The 3'UTR having the secondary structure of (b) may include a nucleotide sequence of any one of SEQ ID NOs: 15 to 17.

The sequence encoding p53 or a functional fragment thereof may be operatively linked to the 5'UTR and the 3'UTR, and may be therefore translated into p53 or a functional fragment thereof.

The p53 or a functional fragment thereof may have activity of preventing or treating cancer. The p53 or a functional fragment thereof may have activity as a tumor suppressor. For example, the tumor suppressor may be involved in regulating the cell cycle of cancer cells themselves, and may accordingly have an anticancer activity. Also, the p53 or a

functional fragment thereof having the anti-cancer activity may suppress proliferation of cancer cells. The p53 or the functional fragment thereof may be a recombinant one including a fusion protein.

The p53 plays an important role in several processes including cell cycle regulation and apoptosis control. A p53 mutation occurs frequently in tumor cells and are related to cancer progression and development of resistance against chemotherapy and radiotherapy. *In vitro* and *in vivo* preclinical studies have shown that recovery of functions of wild-type p53 can induce apoptosis in cancer cells. In animal models having retroviral or adenoviral wild-type p53 constructs, intratumorally injection causes tumor regression in a variety of different tumor tissues, including non-small cell lung cancer (NSCLC), leukemia, glioblastoma, breast cancer, liver cancer, ovarian cancer, colon cancer, and renal cancer.

The sequence encoding a polypeptide may be codon-optimized by encoding the p53 or a functional fragment thereof. The codon optimization may vary depending on a host cell to which the sequence is introduced. Also, the sequence may encode a polypeptide, and may include a degenerate sequence.

A nucleotide sequence encoding a p53 polypeptide may include a sequence encoding an amino acid sequence of SEQ ID NO: 25, for example, a nucleotide sequence of SEQ ID NO: 22.

When the 3'UTR is combined with the 5'UTR, a production yield of RNA may be synergistically improved. When the 3'UTR is combined with the sequence encoding the polypeptide and the 5'UTR, a production yield of RNA may be synergistically improved. In the mRNA, to increase the expression of the p53 or a functional fragment thereof, the 5'UTR having a nucleotide sequence of any one of SEQ ID NOs: 1 to 9 may be combined with the 3'UTR having a nucleotide sequence of any one of SEQ ID NOs: 10 to 17. For example, a combination of the 5'UTR and the 3'UTR may be as follows: 5'UTR of SEQ ID NO: 1 and 3'UTR of any one of SEQ ID NOs: 10 to 17; 5'UTR of SEQ ID NO: 2 and 3'UTR of any one of SEQ ID NOs: 10 to 17; 5'UTR of SEQ ID NO: 3 and 3'UTR of any one of SEQ ID NOs: 10 to 17; 5'UTR of SEQ ID NO: 4 and 3'UTR of any one of SEQ ID NOs: 10 to 17; 5'UTR of SEQ ID NO: 5 and 3'UTR of any one of SEQ ID NOs: 10 to 17; 5'UTR of SEQ ID NO: 6 and 3'UTR of any one of SEQ ID NOs: 10 to 17; 5'UTR of SEQ ID NO: 7 and 3'UTR of any one of SEQ ID NOs: 10 to 17; 5'UTR of SEQ ID NO: 8 and 3'UTR of any one of SEQ ID NOs: 10 to 17; and 5'UTR of SEQ ID NO: 9 and 3'UTR of any one of SEQ ID NOs: 10 to 17. The mRNA may show an increased yield in transcription or translation, compared to mRNA including a C3 3'UTR of SEQ ID NO: 18 or 19 or a P450 2E1 3'UTR. The mRNA may show an increased yield in transcription or translation, compared to mRNA including a C3 5'UTR of SEQ ID NO: 26.

The mRNA may further include an expression regulatory sequence. The expression regulatory sequence may be involved in translational regulation or mRNA stability. The expression regulatory sequence may be a Kozak sequence or a poly (A) sequence.

The nucleotide sequence encoding the 5'UTR, the sequence encoding a polypeptide, the nucleotide sequence encoding the 3'UTR, and the expression regulatory sequence may be transcribed *in vitro* or *in vivo*, and may be form a common transcript.

The nucleotide sequence encoding the 5'UTR, the sequence encoding a polypeptide, the nucleotide sequence encoding the 3'UTR, and the expression regulatory sequence may be operatively linked to one another.

The 5'UTR or the 3'UTR may have activity of increasing the translation yield, stability of the RNA, or a combination thereof, compared to a case where the native 5'UTR and/or the native 3'UTR is used.

In an embodiment, the mRNA may include "5'-cap" such as Cap0, Cap1, or Cap2. The term "5'-cap" refers to a cap structure found at the 5'-end of an mRNA molecule. The 5'-cap is generally a 7-methylguanine ribonucleotide connected to the mRNA via 5' triphosphate at the 5' position of the first nucleotide in a 5'-to-3' chain. In Cap0, riboses at the first and second cap-proximal nucleotides of the mRNA may both include 2'-hydroxyl. In Cap1, riboses at the first and second cap-proximal nucleotides of the mRNA may include 2'-methoxy and 2'-hydroxyl, respectively. In Cap2, riboses at the first and second cap-proximal nucleotides of the mRNA may both include 2'-methoxy. The 7-methylguanine ribonucleotide may be further modified. For example, in the modified 7-methylguanine ribonucleotide, 7-methylguanine 3'-methoxy (7mG(3'OMe)) or 2'-hydroxyl and 3'-hydroxyl may each independently be substituted with a sulfonyl-containing group selected from the group consisting of a mesyl group, an esyl group, a triflyl group, a tresyl group, a tosyl group, a brosyl group, a nosyl group, and a dansyl group. The modified 7-methylguanine ribonucleotide substituted with the sulfonyl-containing group may be, for example, 7-methylguanine 3'-mesyl (7mG(3'OMs)). Most endogenous mRNAs in higher eukaryotes including mammalian mRNA such as human mRNA may have the Cap1 or the Cap2. The cap0 and other cap structures different from the Cap1 and the Cap2 may be immunogenic in mammals such as humans.

Such caps may be introduced co-transcriptionally. For example, the 5'-cap or a 5'-cap analog may be introduced to RNA at initiation, while a DNA template is transcribed *in vitro* in the presence of the 5'-cap or the 5'-cap analog. Also, such caps may be introduced post-transcriptionally. The 5'-cap or the 5'-cap analog may be introduced to RNA post-transcriptionally by a capping enzyme such as a capping enzyme from vaccinia virus. The 5'-end sequence with the 5'-end cap structure formed may include, for example, 7mG(5')ppp(5')ApG, m7G(3'OMe)(5')ppp(5')ApG, m7G(3'OMe)(5')ppp(5')(2'OMeA)pG, m7G (3'OMs)(5')ppp(5')(2'OMeA)pG, or m7G(5')ppp(5')(2'OMeA)pG.

In an embodiment, the mRNA may include a modified nucleotide. In an embodiment, the mRNA may have at least one U substituted with N1-methyl-pseudouridine. In the mRNA, the 5'-end sequence with the 5'-end cap structure formed may have a cap structure of m7(3'OMeG)(5')ppp(5')(2'OMeA)pG or a cap structure of m7(3'OMsG)(5')ppp(5')(2'OMeA)pG. In the mRNA, all Us may be substituted with N1-methyl-pseudouridine. In the mRNA, all Us may be substituted with N1-methyl-pseudouridine, and the 5'-end sequence with the 5'-end cap structure formed may have a cap structure of m7(3'OMeG)(5')ppp(5')(2'OMeA)pG or a cap structure of m7(3'OMsG)(5')ppp(5')(2'OMeA)pG.

The mRNA may include the nucleotide sequence of the 5'UTR any one of SEQ ID NOs: 1 to 9, the nucleotide sequence encoding p53, e.g., the nucleotide sequence encoding an amino acid sequence of SEQ ID NO: 25, and the nucleotide sequence of the 3'UTR of any one of SEQ ID NOs: 10 to 17. The mRNA may include at least one U substituted with N1-methyl-pseudouridine in the sequence. In the mRNA, the 5'-end sequence may have a cap structure of m7(3'OMeG)(5')ppp(5')(2'OMeA)pG or a cap structure of m7(3'OMsG)(5')ppp(5')(2'OMeA)pG. In the mRNA, all Us may be substituted with N1-methyl-pseudouridine. In the mRNA, all Us may be substituted with N1-methyl-pseudouridine, and the 5'-end sequence may have a cap structure of m7(3'OMeG)(5')ppp(5')(2'OMeA)pG or a cap structure of m7(3'OMsG)(5')ppp(5')(2'OMeA)pG. However, the 5'-cap structure of the mRNA is not limited to this structure, and may be substituted with a native 5'-cap structure or a 5'-cap analog known in the art.

The second aspect provides a method of obtaining p53 or a functional fragment thereof, the method including translating the mRNA.

The method may include producing p53 or a functional fragment thereof by culturing a cell including the mRNA.

The third aspect provides a composition including the mRNA as an active ingredient.

The composition may be for delivering, to a subject, the mRNA or p53 or a functional fragment thereof expressed by the mRNA. The delivering may be for preventing or treating cancer in the subject.

The fourth aspect provides a composition for preventing or treating cancer in a subject, the composition including the mRNA as an active ingredient.

The cancer may include a p53 mutation. The cancer may be caused by mutated p53. The cancer may be cancer and/or metastasis thereof that can be treated, reduced, inhibited, ameliorated, and/or prevented by supplementation of wild-type p53 or a functional fragment thereof.

The composition may be for treating, reducing, inhibiting, ameliorating, and/or preventing cancer and/or metastasis thereof in a subject. The subject may be a mammal including a human.

The composition may be administered to the subject, and RNA (e.g., mRNA) may be then translated *in vivo* to produce a therapeutic polypeptide, i.e., p53 or a functional fragment thereof.

The composition may be brought in contact with a cell, a tissue, or a subject in an "effective amount" of the mRNA.

The effective amount may be determined based on a target tissue, a target cell, a means of administration, physical properties of RNA (e.g., size of RNA and an amount of modified nucleoside), and other components of the composition.

The composition may be administered by intramuscular, subcutaneous, intradermal, intranasal, or pulmonary administration.

The composition may include at least one pharmaceutically acceptable carrier or an excipient. In the composition, RNA may be formulated with or in complex with the carrier or the excipient. The carrier or the excipient may be one known in the art.

Relative amounts of active ingredients, pharmaceutically acceptable carriers, excipients, and/or other additional components included in the composition may vary depending on the identity, size and/or conditions of the subject to be treated, and/or routes of administration. For example, the composition may include 0.1 % to 100 %, for example, 0.5 % to 50 %, 1.0 % to 30 %, 5.0 % to 80 %, or 80 %(w/w) or more of active ingredients.

The composition may be formulated as nanoparticles. The nanoparticles may be those known in the art for delivering a polynucleotide, such as an mRNA, into a cell. For example, the nanoparticles may be those known to be used in an RNA vaccine. The nanoparticles may be lipid nanoparticles (LNPs). The composition may be formulated as or bound to LNPs. The binding may include binding with LNPs or binding to the surface of LNPs. For example, the composition may be formulated inside a lipid polycation complex, or may be bound to a lipid polycation complex. The lipid polycation complex is also known as a cationic lipid nanoparticle. The polycation may include MC3, Lipid 319, C12-200, 5A2-SC8, 306Oi10, Moderna Lipid 5, Acuitas A9, SM-102, ALC-0315, Arcturus Lipid 2,2 (8,8) 4C CH3, Genevant CL1, or cationic polypeptides such as polylysine, polyornithine, and/or polyarginine. Also, the composition may be formulated with or bound to LNPs including 1,2 distearoyl-sn-glycero-3-phosphocholine (DSPC), dipalmitoylphosphatidylcholine (DPPC), sterol such as cholesterol, or a non-cationic lipid such as dioleoylphosphatidylethanolamine (DOPE). Also, the composition may be formulated with or bound to LNPs including a polyethyleneglycol (PEG)-lipid such as 1,2-dimyristoyl-rac-glycero-3-methoxypolyethylene glycol-2000 (PEG2000-DMG), 2-[(polyethylene glycol)-2000]-N,N-ditetradecylacetamide (ALC-0159), and polyethylene glycoldimethacrylate (PEG-DMA).

The LNP formulation may include cationic lipids, phospholipids, sterols such as cholesterol, PEG-lipids, or a combination thereof. The LNP formulation may include, for example, cationic lipids, phospholipids, sterols such as cholesterol, and PEG-lipids. Also, the LNPs may include PEG-modified lipids, non-cationic lipids, sterols, ionizable lipids, or a combination thereof. The LNPs may include 0.5 to 15 mol% of PEG-modified lipids, 5 to 25 mol% of non-cationic lipids, 25 to 55 mol% of sterols, and 20 to 60 mol% of ionizable lipids. The PEG-modified lipid may be PEG2000-DMG, the non-cationic lipid may be DSPC, the sterol may be cholesterol, and the ionizable cationic lipid may be Compound 1 having the following structure:

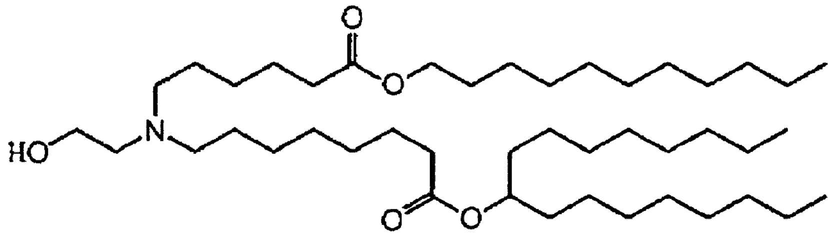

(Compound 1).

The PEG-modified lipid may be Compound 2 (ALC-0159) having the following structure, the non-cationic lipid may be DSPC, the sterol may be cholesterol, and the ionizable cationic lipid may be Compound 3 (ALC-0315) having the following structure:

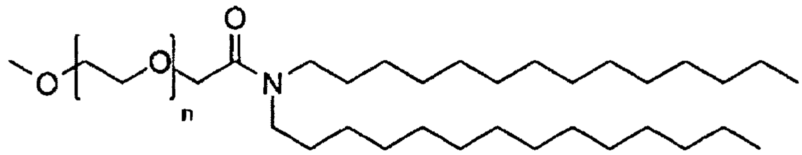

(Compound 2);

and

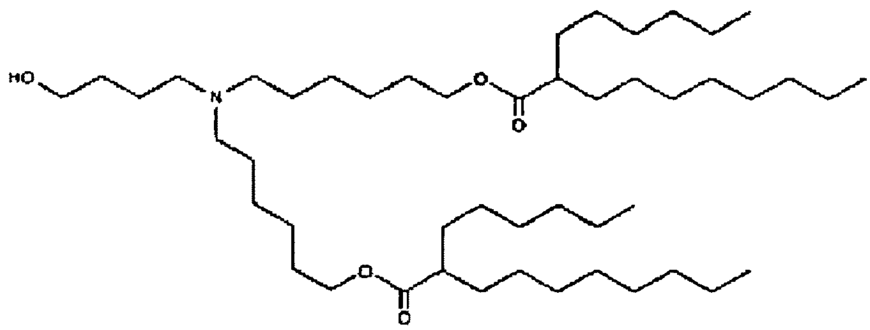

(Compound 3).

The composition may be a composition for preventing or treating cancer in a subject including, as active ingredients, the mRNA and the LNPs.

The fifth aspect provides a method of utilizing the mRNA, the method including introducing the mRNA to a host cell.

In the method, the host cell may be any cell derived from a subject. The subject may be a mammal including a human. The cell may be, for example, any cancer cell.

The method may include administering the mRNA into a subject. The administering may be parenteral or oral administration. The administering may be intramuscular, subcutaneous, intradermal, intranasal, or pulmonary administration. The subject may be a mammal including a human.

The method may be for inducing prevention or treatment of cancer in the subject. The cancer is the same as described above.

In the method, the administering may be intramuscular, subcutaneous, intradermal, intranasal, or pulmonary administration.

The administering may be carried out in an "effective amount" for preventing or treating cancer.

The sixth aspect provides DNA encoding the mRNA.

The DNA may further include a sequence for introducing a transcribable nucleotide sequence. The sequence for introducing a transcribable nucleotide sequence may be operatively linked to a 3'UTR upstream of a nucleotide encoding the 3'UTR. The sequence for introducing a transcribable nucleotide sequence may be a cloning site. The cloning site may be a multiple cloning site. The cloning site may include a recognition site for a restriction enzyme, a cleavage site, or a

combination thereof.

The DNA may further include a promoter sequence. The promoter may be operatively linked to a 5'UTR upstream of a nucleotide encoding the 5'UTR.

The promoter, the nucleotide sequence encoding the 5'-UTR, the sequence encoding a polypeptide or the nucleotide sequence for introducing a transcribable nucleotide, and the nucleotide sequence encoding the 3'UTR may be linked to one another to form a common transcript when transcription occurs *in vitro* or *in vivo.*

The DNA may be a nucleic acid construct or a vector. The DNA may be an expression vector or a cloning vector.

The seventh aspect provides a method of obtaining RNA, the method including transcribing RNA by using the DNA as a template. The transcribing may include *in vitro, ex vivo,* or *in vivo* transcription. The *in vivo* transcription may include transcription occurring in a subject.

The term "*in vitro* transcription" or "RNA *in vitro* transcription" refers to a process of synthesizing RNA including an mRNA in a non-cell system, that is, *in vitro.* The cloning vector DNA including a plasmid DNA vector may be applied as a template for generating an RNA transcript. These cloning vectors are also generally known as transcription vectors. RNA may be obtained by DNA-dependent *in vitro* transcription with a suitable DNA template. The DNA template may be a linearized plasmid DNA template. The promoter controlling RNA *in vitro* transcription may be any promoter for a DNA-dependent RNA polymerase. The DNA-dependent RNA polymerase may be a T7 RNA polymerase, a T3 RNA polymerase, a SP6 RNA polymerase, or a combination thereof. The DNA template for RNA *in vitro* transcription may be obtained by cloning a nucleic acid and introducing the same to a vector for RNA *in vitro* transcription. The DNA may include cDNA corresponding to each RNA to be transcribed *in vitro.* The vector for RNA *in vitro* transcription may be circular plasmid DNA. The cDNA may be obtained by reverse-transcription of an mRNA or by chemical synthesis.

The transcription vector may include a promoter, a nucleotide sequence encoding the 5'-UTR, a sequence encoding a polypeptide, such as an ORF, a nucleotide sequence encoding the 3'-UTR, and a poly (A) tail. The transcription vector may also include a replication origin, such as *E. coli* replication origin ColE1 ori, a selection marker gene, or a combination thereof. The promoter may be a T7 promoter. The selection marker may be an antibiotic resistance enzyme, such as a kanamycin resistance enzyme.

The *in vitro* transcription may include obtaining a transformed host cell by introducing the transcription vector to a host cell, for example, *E. coli*, and proliferating the host cell, which includes the plasmid DNA that becomes a template for an mRNA, by culturing the host cell. The *in vitro* transcription may include isolating the plasmid DNA from the proliferated host cell, for example, *E. coli*. The isolating of the plasmid DNA may include isolating a cell layer from the culture and isolating the plasmid DNA from the cell layer. The isolating of the cell layer may be performed by centrifugation, separation, precipitation, or a combination thereof. Isolation of plasmid DNA from cells may be performed by methods known in the art, such as an alkali extraction method, affinity chromatography, high performance liquid chromatography (HPLC), electrophoresis, or a combination thereof.

The obtaining of RNA may include producing an RNA transcript of the DNA by culturing the cell including the DNA.

## Advantageous Effects of Invention

The mRNA according to an aspect is relatively stable and has a high production yield, and thus may be used to transcribe and translate an mRNA efficiently.

According to the method of obtaining a polypeptide in accordance with another aspect, p53 and a functional fragment thereof may be obtained efficiency.

The composition for delivering the mRNA or p53 expressed thereby to a subject according to another aspect may be used to deliver the mRNA or p53 expressed thereby to a subject.

The composition for preventing or treating cancer in accordance with another aspect may be used to immunize or treat a subject efficiently.

The method of using RNA according to another aspect may be used to prevent or treat cancer in a subject.

The DNA according to another aspect may be used to obtain the mRNA.

According to the method of obtaining RNA in accordance with another aspect, RNA may be obtained efficiently.

## Brief Description of Drawings

FIG. 1 is a diagram showing results of confirming inhibitory ability of the produced p53 mRNA on growth and development in each cancer cell.

FIG. 2 is a diagram showing changes in tumor size and body weight, when a p53 mRNA anti-cancer candidate substance is administered to an ovarian cancer xenograft mouse model.

FIG. 3 is a diagram showing changes in tumor size and body weight, when a p53 mRNA anti-cancer candidate

substance is administered to a pancreatic cancer xenograft mouse model.

FIG. 4 is a diagram showing changes in tumor size and body weight, when a p53 mRNA anti-cancer candidate substance is administered to a lung cancer xenograft mouse model.

## Best Mode for Carrying out the Invention

Hereinafter, the present disclosure will be described in more detail through examples. However, these examples are intended to illustrate the present disclosure, and the scope of the present disclosure is not limited to these examples.

### Example 1: Unstructured 5'UTR non-natural sequence increasing yield of genes

In this example, 5'UTR sequences increasing a yield of genes were selected. A 5'UTR sequence is a non-natural sequence that does not exist in nature, and is not a sequence that forms a secondary structure. The 5'UTR sequence has activity of increasing a yield of genes, compared to the native sequences.

First, random non-natural sequences with a length of 50 bp or 70 bp and a mean free energy of 0 were selected, and sequences that did not form a secondary structure between bases were selected.

The selected mRNA sequences were designed to start with 'AG' for capping optimization to protect the 5'-end and end with 'GCCACC' which is a Kozak sequence for stable gene expression.

The sequences having an excellent activity of increasing the yield of genes were first selected. Based on these sequences, some base sequences were substituted to improve the activity in increasing the yield of genes, and therefore 9 non-natural sequences were finally selected (Table 1).

[Table 1]

| SEQ ID NO: | 5'UTR sequence |
|---|---|
| 1 | AGCCGUCUUCCAUCGUUACAGUCACCGUUUUCGCAUUCUGUCUA GCCACC |
| 2 | AGAAUCCAGUCAACGCAUAUCCACGUAUACCCGAACACAUCACAG CCACC |
| 3 | AGCCCCCCAACACUGACUUAAUCGCUUUAAGUUCCUCCAAACGAG CCACC |
| 4 | AGAGCAGAACGCGUAACCACACCAUCGCAACAUCCCACUUGAUAG CCACC |
| 5 | AGACCAGACACCUAACAGACUAGACAUUUUACCGAAAUCCGCAUU AACCAUUCUAACUCGUAUAGCCACC |
| 6 | AGAUCACUAACACCAUAUAACGCUAUCUUCCUCCUUCUCCGACAU UCCAGUACUCAUUCCACAAGCCACC |
| 7 | AGUACCCACUCCAGAUAAACUGACAACCAUACGCGAACAACGACG AAUCGUAUCACACGCAGAAGCCACC |
| 8 | AGCGAGAUAACUUACAAGGAGAGAGAAGACGAGGAGAGAUAAAUU AGAGAGAAAACGAGUAAGAGCCACC |

(continued)

| SEQ ID NO: | 5'UTR sequence |
|---|---|
| 9 | AGUAAACUAACCACUCCAUCACGUAUAUAUUCCUCUUUCACACCU UUCCAUUCUCGAACUAACCGCCACC |

Each nucleotide in the nucleotide sequences of SEQ ID NOs: 1 to 9 may be modified from naturally occurring nucleotides. For example, at least one U in the sequence may be substituted with N1-methyl-pseudouridine. In the sequence, all Us may be substituted with N1-methyl-pseudouridine.

**Example 2: Specifically-structured 3'UTR non-natural sequence increasing yield of genes**

In this example, non-natural 3'UTR sequences that form a structure similar to the natural 3'UTR structure having excellent activity in increasing the yield of genes and that have activity in increasing the yield of genes compared to the native 3'UTR structure were selected.

As the native 3'UTR, a human complement component 3 (C3) 3'UTR (SEQ ID NO: 18) or a human cytochrome P450 2E1 3'UTR (SEQ ID NO: 19), which is known to have an excellent activity of increasing the yield of genes, was used. Specifically, random sequence combinations having the same length as the human C3 3'UTR or the human cytochrome P450 2E1 3'UTR were constructed, and sequences of which secondary structures were similar to the human C3 3'UTR or the cytochrome P450 2E1 3'UTR were selected.

Regarding the selected mRNA sequences, a process of removing potential AU-rich element (hereinafter referred to as 'ARE') sequences that can cause mRNA unstable was performed, and then, 8 non-natural 3'UTR sequences were finally selected (Table 3).

Native C3 3'UTR sequence (SEQ ID NO: 18-length 178 nt):

CCACACCCCCAUUCCCCCACUCCAGAUAAAGCUUCAGUUAUAUCUCACGU
GUCUGGAGUUCUUUGCCAAGAGGGAGAGGCUGAAAUCCCCAGCCGCCUCACCU
GCAGCUCAGCUCCAUCCUACUUGAAACCUCACCUGUUCCCACCGCAUUUUCUCC
UGGCGUUCGCCUGCUAGUGUG

Secondary structure of native C3 3'UTR sequence (represented by dot-bracket notation):

.

(((((...........(((...(((((.(((....))).)))))...)))..(((((((..((((....((((((.(((((.......)))))..))-
).)))))))...)))) )))........(((....)))............(((.........(((....))))))..)))))

The non-natural sequences (SEQ ID NOs: 10 to 14) having a structure similar to the structure of the native C3 sequences were designed to have a GC% of 40 to 50 % and a mean free energy for RNA folding of -0.1 to -0.3 kcal/mol.

Native cytochrome P450 2E1 3'UTR sequence (SEQ ID NO: 19-length 158 nt):

GUGUGUGGAGGACACCCUGAACCCCCCGCUUUCAAACAAGUUUUCAAAU
UGUUUGAGGUCAGGAUUUCUCAAACUGAUUCCUUUCUUUGCAUAUGAGUAUUU
GAAAAUAAAUAUUUUCCCAGAAUAUAAAUAAAUCAUCACAUGAUUAUUUUAACUA
U

Secondary structure of native cytochrome P450 2E1 3'UTR sequence
((((((((((((...(((((.((........((((((((.........))))))))))))))).....(((...))).....)))))))))))..(((((((((((....) )))))...))))))......((((((...))))))...........

The non-natural sequences (SEQ ID NOs: 15 to 17) having a structure similar to the structure of the native human cytochrome P450 2E1 3'UTR sequence were designed to have a GC% of 30 to 40 % and a mean free energy for RNA folding of -0.1 to -0.3 kcal/mol.

[Table 2]

| SEQ ID NO: | 3'UTR sequence |
|---|---|
| 10 | ACCACCAAGACAAAAACCACGAAUUUACGCCAAAAGUGGAGUAAGGAG GUGCGUCCCUUACGCAACACGACAGCCGGGAUCUAACAAUAGGGUUA AUGGACUGGUUGAGAUAAGGGGCAUUAAAACCAAACGGUCUACACAUA AAACGAAUGAAAACUUCCUAAAGGAUCGACGGUGG |
| 11 | CAUACCAUAAAACCAAACAUAUACCCGAGGUCCUUCGGCAUUGGGAGG AUGAUACGCAAGAAGCAUGAUUCCCCACCAGACCGAAUGCAGGUUUAA GUGAGGGGUGCAAACUUGUGUCACUAACCAUUAUUAUGAAGAAAAAAA CAGCCAAAAAAAGAGCCACAAGGCGGCGCGGUAU |
| 12 | GCCGCUAAAUAAACGAACACGUACCUACGCUUUGACAGGAGUGGGCA AGUGGGAAGGUUACCGGAACUACGAGGACCACCAGGCAAGUAUUGGU UAGUCACUCUUCCAACUAAUCUUAAUAAAACGAGACAUCGAAAAAAAAA AAAGGCAAAAACAAGUCCAGAAGGAGCCAGGGUGG |
| 13 | AGAGUAAAAUUAAUAUACGGUAUAGAACUCACCAUUGUGCGUUCUACC UCGAGGUGUCAUGACGACAGUGUCCCAUAUCAACCCGAGAAGUUGGA AAUGCGGAGUCGAACGUGACGCCAAUAAGGCCAAAAGGUAAAUAAUGA AACGUACAACCGAAUGCCAAAAGGCUACGCUGCUC |
| 14 | GUACAUGCUACAAACCAUGCGGAACCUACAAGACACUUUAUGGGUAAA GCAACAAACCCACUUGUAAAGUUACCACACCGGUCCCAUCUGCCGGCC GUGAGUAUAUGAUCUGGGUUUAUAAUCUCGAAAUAUCGCGAAACAAUA UGCCCUUAGAAUAAGCACCCAUGCGGGCAAUGUA |
| 15 | UCUUUAUGAGAAUUACUCAUUCUGAUUUUUAACAUAGCAUUUUUUUUC UGUUGUGUAGAUGGGCCAAAGGACAUUCCAUAACUUUUCAUAAGGAC AAACGAUUACCGACGGAUUGGUACUAGUUGUUAAUUAACAUCUGGGG GAUGUAAACAUUUUUU |
| 16 | UACGAACUGAAAUAAUCAAGUGAUAGUUAUCAAUUAAAAGCUUUAUAA UUUUAGUUUCCUUGGGCCACGGAAUUUCCACAAUUUUUAGUUCGUAA ACCGGUCAUCCAAUCUAUUGGGUUGAGGCUGGAAUAUGCACCUUAUG GGUGUCUUUGUAUAAA |

(continued)

| SEQ ID NO: | 3'UTR sequence |
|---|---|
| 17 | AACACAGAAAACUACCACUAUACUUCUAUUUAGAAAGCACUUAUUAAUUGCUUUUUGUUAGUGAAACAUGCAAGGCACCCAUGUUUUCUGUGUUAACCAAGCCAGUUGAUAUUAGCUGUAUGCUUGGAUUCUAUACAUAAAAUGUAUUUAAGCAGCUC |

The RNA folding energy provides insights into the secondary structure and stability thereof. For example, the minimum free energy may be used to predict the secondary structure of 3'UTR and stability thereof, which may affect the translation yield.

**Example 3: Preparation of template vector using non-natural 5'-UTR sequence and 3'-UTR sequence**

In this example, a vector prepared by operably linking: one of the sequences encoding the non-natural 5'UTR nucleotide sequence including the nucleotide sequences of SEQ ID NOs: 1 to 9 obtained in Examples 1 and 2 or the C3 5'UTR of SEQ ID NO: 26; one of the sequences encoding the 3'UTR nucleotide sequence including the nucleotide sequences of SEQ ID NOs: 10 to 17 or the C3 3'UTR of SEQ ID NO: 18 or 19 or the P450 2E1 3'UTR; and the gene encoding the polypeptide (wild-type p53 protein, SEQ ID NO: 25).

Specifically, the vector is one in which a promoter, a nucleotide sequence encoding 5'-UTR, an open reading frame (ORF) encoding a polypeptide, a nucleotide sequence encoding 3'-UTR, and a poly (A) tail.

The vector may also include *E. coli* replication origin ColE1 ori and a selection marker gene. The promoter may be a T7 promoter. The selection marker may be a kanamycin resistance enzyme.

**3-1. Preparation of template vector for transcription of native p53 and p53 mRNA**

Native p53 mRNA and/or a protein expression vector thereof was prepared, including one of nucleotides encoding the designed non-natural 5'UTR sequence, a nucleotide sequence, as an ORF, encoding the native p53 protein, one of nucleotide sequences encoding the non-natural 3'UTR, and a poly (A). This vector was introduced to a microbial cell, and a cloning vector was produced by culturing the microbial cell. Through *in vitro* transcription with the produced vector as a template, mRNA was produced.

Specifically, a vector pUC57-Kan$^R$, in which an antibiotic resistance gene was substituted with a kanamycin resistance gene, was produced from the pUC57-Amp$^R$ vector (Addgene). The produced pUC57-Kan$^R$ vector was treated and cleaved with restriction enzymes HindIII and EcoRI, and the cleaved vector was connected to a synthetically produced polynucleotide encoding a native p53 protein by using a ligase to insert a sequence encoding a native p53 protein. Since the inserted polynucleotide encoding a native p53 protein includes a nucleotide sequence encoding 5'UTR and a sequence encoding a native p53 protein, and restriction enzyme recognition sequences are at the 3'-end of the native p53 nucleotide sequence, the restriction enzyme recognition sequences were cleaved to introduce a nucleotide sequence encoding the designed 3'UTR.

[Table 3]

| SEQ ID NO: | Gene sequence (DNA) of template vector for transcription of native p53 mRNA |
|---|---|
| 20 | TAATACGACTCACTATA |
| 21 | **AGTGCTCCCCATCCAACTAAACTGTCCCTCTGTCCGAACTAAACTGCACTGTCCCAGCACC** |

(continued)

| SEQ ID NO: | Gene sequence (DNA) of template vector for transcription of native p53 mRNA |
|---|---|
| 22 | ATGGAAGAACCTCAGTCCGATCCGAGCGTCGAGCCTCCCCTGTCACAG<br>GAAACTTTCAGTGACCTGTGGAAACTGCTGCCCGAGAATAATGTACTTT<br>CGCCTCTGCCTAGCCAAGCAATGGATGATCTGATGCTGTCTCCAGATGA<br>CATCGAACAGTGGTTTACAGAGGATCCAGGGCCCGATGAGGCACCACG<br>AATGCCAGAGGCGGCCCCACCTGTTGCACCCGCACCTGCGGCTCCAAC<br>ACCCGCAGCCCCCGCCCCCGCTCCAAGCTGGCCTCTCAGTAGTTCCGT<br>GCCATCACAGAAGACCTATCAAGGCAGCTACGGATTTCGCCTCGGATTT<br>CTCCATTCAGGCACTGCCAAGTCCGTGACTTGTACGTACAGCCCCGCT<br>CTGAACAAAATGTTCTGTCAGTTGGCGAAGACATGTCCTGTCCAGCTTT<br>GGGTAGATTCTACCCCCCCGCCGGGGACACGGGTGCGAGCCATGGCC<br>ATTTATAAACAGTCTCAGCACATGACCGAGGTCGTGCGCAGGTGTCCTC<br>ACCATGAACGATGCTCCGACTCCGACGGCCTGGCCCCCCCACAGCACC<br>TGATTCGCGTGGAAGGCAATCTTCGTGTGGAGTACCTCGATGATCGGA<br>ACACCTTCAGACATAGCGTGGTTGTGCCTTATGAGCCACCCGAAGTTGG<br>AAGCGACTGTACCACCATCCATTACAACTATATGTGCAACAGTAGCTGC<br>ATGGGTGGCATGAATCGGCGGCCTATCTTGACAATAATCACTTTAGAGG<br>ACTCTTCCGGAAACCTGCTTGGCAGAAATTCATTCGAGGTCCGTGTCTG<br>CGCCTGCCCGGGCAGGGACCGCAGAACCGAAGAGGAGAATTTACGGA<br>AAAAGGGGGAACCCCACCACGAACTGCCACCGGGCAGTACCAAAAGG<br>GCTCTCCCTAATAACACATCTTCATCGCCCCAGCCAAAGAAGAAACCCC<br>TCGACGGAGAGTACTTTACTCTACAAATTAGGGGGAGAGAAAGATTTGA<br>GATGTTCCGCGAATTGAACGAGGCCCTGGAGTTGAAAGACGCTCAAGC<br>CGGAAAGGAACCAGGTGGGTCTAGGGCCCACTCAAGCCATCTGAAGAG<br>TAAGAAAGGCCAGTCCACTTCCAGGCACAAAAAGTTAATGTTCAAGACG<br>GAGGGTCCTGACTCTGAC |
| 23 | GTGTGTGGAGGACACCCTGAACCCCCCGCTTTCAAACAAGTTTTCAAAT<br>TGTTTGAGGTCAGGATTTCTCAAACTGATTCCTTTCTTTGCATATGAGTA<br>TTTGAAAATAAATATTTTCCCAGAATATAAATAAATCATCACATGATTATT<br>TTAACTAT |

(continued)

| SEQ ID NO: | Gene sequence (DNA) of template vector for transcription of native p53 mRNA |
|---|---|
| 24 | AAAAAAAAAAAAAAAAAAAAAAAAAAAAAAAAAAAAAAAAAAAAAAAAAAA<br>AAAAAAAAAAAAAAAAAAAAAAAAAAAAAAAAAAAAAAAAAAAAAAAAAAA<br>AAAAAAAAAAAAAAAAAA |

Table 3 shows a promoter (SEQ ID NO: 20), a polynucleotide encoding 5'UTR (SEQ ID NO: 21), a polynucleotide encoding a native p53 protein (SEQ ID NO: 22), a polynucleotide encoding 3'UTR (SEQ ID NO: 23), and a poly (A) sequence (SEQ ID NO: 24), included in the prepared pUC57-KanR vector. In Table 3, the underlined nucleotides in SEQ ID NO: 20 represent a portion of a T7 promoter involved in the initiation of mRNA transcription. Also, the bolded nucleotides of SEQ ID NO: 21 represent 5'UTR sequences derived from the human C3 native 5'UTR sequences. Also, SEQ ID NO: 22 is, as a 5'UTR downstream nucleotide sequence, a nucleotide sequence encoding a native p53 protein. The native p53 protein has an amino acid sequence of SEQ ID NO: 25. Also, SEQ ID NO: 23 is a native 3'UTR sequence of the human cytochrome P450 2E1 gene.

### 3-2. Connection of non-natural 5'UTR sequence to nucleotide sequence encoding native p53 protein and cloning

To substitute the 5'UTR sequence of the prepared template vector with a nucleotide sequence encoding a non-natural 5'UTR sequence, a DNA fragment containing the nucleotide sequence encoding the 5'UTR was obtained through a PCR reaction with a primer set of a primer including the nucleotide sequence encoding the non-natural 5'UTR sequence and a primer including the sequence of the XhoI restriction enzyme recognition sequence of the native p53 protein, and with the template vector as a template.

Each of DNA fragments containing the nucleotide sequences encoding 5'-UTRs secured by PCR reaction, and the template vector prepared in Example 3-1 were treated and cleaved with restriction enzymes HindIII and XhoI, and the cleaved sequences were connected by using a ligase. As a result, a vector encoding the native p53 protein, in which the nucleotide sequence encoding 5'UTR is substituted with the nucleotide sequence encoding non-natural 5'UTR of any one of SEQ ID NOs: 1 to 9, was prepared.

### 3-3. Cloning of non-natural 3'UTR sequence

In this example, the non-natural 3'UTRs of SEQ ID NOs: 10 to 17 were cloned to increase the expression level and to increase the sequence stability of the gene encoding mRNA. The 3'UTR may include XhoI (CTCGAG) and NheI (GCTAGC) restriction enzyme sequences at the upstream 5'-end and the downstream 3'-end, respectively, to be used for recombination of a nucleic acid such as the transcribable nucleic acid sequence or a polyadenyl sequence. The transcribable nucleic acid sequence may be an ORF. The 3'UTR sequence may be linked to the 3'-end of the ORF via the XhoI restriction enzyme at the 5'-end present in the transcribable nucleic acid sequence.

The recombinant 3'UTR sequence including the XhoI restriction enzyme sequence, the non-natural 3'UTR sequence, and the NheI restriction enzyme sequence was synthesized by an in vitro nucleic acid synthesis method. The obtained recombinant 3'UTR sequence is DNA. Each of the recombinant 3'UTR sequence and the template vector was treated and cleaved with restriction enzymes XhoI and NheI, and both the cleaved sequences were connected by using a ligase. For use as a template vector, the vector used in Examples 3-1 and 3-2 was used.

As a result, a vector for p53 expression was prepared, in which a T7 promoter-5'UTR-coding nucleotide sequence-p53 protein-coding sequence-3'UTR-coding nucleotide sequence-poly (A) structure was introduced into the backbone of the pUC57-Kan$^R$ vector.

### Example 4: Production of gene products using non-natural 5'-UTR sequence and 3'-UTR sequence

The vector prepared in Example 3, having a T7 promoter-5'UTR-coding nucleotide sequence-p53 protein-coding sequence-3'UTR-coding nucleotide sequence-poly (A) structure, was introduced into *E. coli*, and the *E. coli* was cultured to proliferate the *E. coli* including a plasmid DNA as a template for the mRNA. The proliferated *E. coli* was isolated from the culture by high-speed centrifugation, and the cell-derived template DNA was secured by using the alkali extraction method known in the art. The secured template DNA was linearized by using the restriction enzyme recognition sequence

located at the end of the poly (A) tail. mRNA having nucleotide sequences with a 5'UTR-p53 protein-coding sequence-3'UTR-poly (A) structure was produced via in vitro transcription using the linearized cell-derived template DNA. Here, 5'UTR has a sequence of any one of SEQ ID NOs: 1 to 9 or a sequence of native C3 5'UTR, the 3'UTR has a sequence of one of SEQ ID NOs: 10 to 17 or a sequence of native C3 3'UTR or native CYP 2E1 3'UTR, and the sequence encoding a p53 protein has a sequence of SEQ ID NO: 22. The sequence of SEQ ID NO: 22 is one of nucleotide sequences encoding a p53 protein of SEQ ID NO: 25, and any nucleotide sequence that can encode a p53 protein may be used.

Specifically, to an aqueous solution containing 10 ng/μl of the linearized plasmid DNA, 4 mM of a capping reagent, 5 mM of ATP, 5 mM of CTP, 5 mM of GTP, 5 mM of N1-methylpseudouridine-5'-triphosphate, 20 mM of magnesium ion salt, 10 mM of DTT, 0.01 U/μL of inorganic pyrophosphatase (PPase), and 1 U/μL of RNAase inhibitor, 5 U/μL of T7 RNA polymerase was added, and the reaction mixture thus obtained was incubated at 37 °C for at least one hour, to proceed in vitro transcription. For use as the capping reagent, the cap analog compound m7G(3'OMs)pppA(2'OMe)pG of Example 8 described in 10-2023-0141482 published on October 10, 2023 was used.

The capping reagent is a reagent for mRNA transcription and co-transcriptional capping, and forms a 5'-cap structure of m7G(3'OMs)pppA(2'OMe)pG. Here, m7G represents 7-methylguanosine, A represents adenosine, G represents guanosine, p is - P(=O)(OH)O-, Ms represents mesyl, and Me represents methyl. The reaction product was treated with a DNase, and RNA was purified by using a Monarch® RNA Cleanup Kit (New England Biolabs).

As a result, a vector for p53 expression was prepared, in which a T7 promoter-5'UTR-coding nucleotide sequence-p53 protein-coding sequence-3'UTR-coding nucleotide sequence-poly (A) structure was introduced into the backbone of the pUC57-Kan$^R$ vector.

### Example 5: Confirmation of effects on cell survival in cancer cells having a p53 mutation

The effects of the p53 mRNA prepared in Example 4 on cancer cells having a p53 mutation was confirmed. The p53 mRNA used herein has a 5'UTR(SEQ ID NO: 6)- p53 protein-coding sequence (SEQ ID NO: 25)- 3'UTR(SEQ ID NO: 11)- poly (A) (SEQ ID NO: 24) structure (SEQ ID NO: 27). For use as cancer cells having p53 mutation, ES-2(ATCC, #CRL-1978), Mia-PaCa-2(ATCC, #CRL-1420), H1299(Korean cell line bank, #KCLB25803), and SNU-1066(Korean cell line, #KCLB01066). The ES-2 is a p53 S241F mutant ovarian cancer cell line, and shows fibroblast-like morphology isolated from the ovary of a black female with clear cell carcinoma. Mia-PaCa-2 is a p53 R248W mutant pancreatic cancer cell line, and is an epithelial cell line derived from tumor tissue of the pancreas of a white male. H1299(NCI-H1299) is a lung cancer cell line with a p53 deletion mutation. It is an epithelial-like cell isolated from a white male, cancer lung patient, and may belong to the large cell carcinoma subtype of non-small cell lung cancer. SNU-1066 is a p53 514_559del46, as a mutant head and neck cancer cell line, is a laryngeal squamous cell carcinoma cell line.

Specifically, by using Lipofectamine™ MessengerMAX™ transfection reagent (Thermo Scientific™, Cat No. LMRNA001), the p53 mRNA was delivered to each cancer cell line, and the effect on cell survival was confirmed. In a 96-well plate, ES-2 cells, Mia-PaCa-2 cells, H1299 cells, and SNU-1066s were seeded at $3x10^3$ cells/200 μL per well, $6x10^3$ cells/200 μL per well, $5x10^3$ cells/200 μL, per well, and $10x10^3$ cells/200 μL per well, respectively. 24 hours after seeding, mRNA and 0.2 μL of Lipofectamine™ MessengerMAX™ transfection reagent were added to each well. Then, p53 mRNA was tested at 8 concentrations, starting from 4 μg/mL and decreasing by 1/4.

The cell viability was measured by using a WST-8 cell viability assay kit (BIOMAX, Cat No. QM5000) reagent. To determine cell growth, the day of drug treatment was set as Day 0, and the WST-8 reagent was mixed with a medium at a ratio of 1:10 in cells seeded separately the day before and reacted for 2 hours, and then the absorbance at a wavelength of 450 nm was measured. The drug-treated plate was cultured at 37 °C for 3 days. After completion of the culture, the WST-8 reagent was mixed with a cell medium at a ratio of 1:10 and reacted for 2 hours. The absorbance at a wavelength of 450 nm was measured, and the half maximal growth inhibition concentration ($GI_{50}$) values were calculated by using a Graphpad Prism™ software. The relative cell growth rate, compared to the negative control group in which the p53 mRNA was not treated, was derived by correcting the value at Day 0 and the blank OD value.

As a result of confirming the growth inhibition of ability of the p53 mRNA in each cell, it was confirmed that the p53 mRNA inhibited the growth of ovarian cancer, pancreatic cancer, lung cancer, and head and neck cancer cell lines with defects in the p53 gene (FIG. 1).

### Example 6: Evaluation of anticancer efficacy of p53 mRNA anticancer candidate agent in ovarian cancer xenograft model

To evaluate the anticancer efficacy of the p53 mRNA according to the present disclosure, the p53 mRNA was administered to an ovarian cancer mouse model in which the ES-2 cells were xenografted, and changes in tumor size were observed. To the administration group, a p53 mRNA anticancer therapeutic agent was administered three times a week (7.5, 15, and 30 μg/head) for 4 weeks.

Specifically, the ES-2 cells (ATCC) were injected subcutaneously into the dorsal side of a 5-week-old BALB/c

nude mouse. When tumors were observed with the naked eye a few days later, the size of the tumors was then monitored. At the beginning of administration, 6 mice having tumors in similar size were assigned to each group. Here, the administration group was administered intravenously. During the experimental period, the body weight of the mice and the tumor size were measured 2 to 3 times a week. The tumor size was measured according to the equation below, by measuring a longest length and a longest width with a digital caliper.

$$\text{Tumor size (mm}^3\text{): Width (mm) x Width (mm) x Length (mm) / 2}$$

The tumor size of each group was observed until Day 21 starting from the starting day of the administration, and the calculation was applied in the same manner for the tumor size measured in each following experiment.

When administered to the ovarian cancer xenograft model mouse, the p53 mRNA anticancer therapeutic agent not only showed significant tumor growth inhibition efficacy in the ES-2 xenograft model compared to the control group (administered with empty LNP as a vehicle) (FIG. 2A), but also appeared to be safe in terms of side effects with no decrease in the body weight of the mouse after 3 weeks of continuous mRNA administration (FIG. 2B). On Day 21 after administration, the tumor inhibition ability was calculated to 54.8 %, 62.8 %, and 78.9 % in the in 7.5 μg, 15 μg, and 30 μg administration groups, respectively, confirming that the anticancer efficacy was dose-dependent.

**Example 7: Evaluation of anticancer efficacy of p53 mRNA anticancer candidate agent on pancreatic cancer xenograft model**

To evaluate the anticancer efficacy of the p53 mRNA according to the present disclosure, the p53 mRNA was administered to a pancreatic cancer mouse model in which the Mia-PaCa-2 cells were xenografted, and changes in tumor size were observed. To the administration group, a p53 mRNA anticancer therapeutic agent was administered three times a week (6 times in total, 30 μg/head) for 4 weeks.

Specifically, the Mia-PaCa-2 cells (ATCC) were injected subcutaneously into the dorsal side of a 5-week-old BALB/c nude mouse. When tumors were observed with the naked eye a few days later, the size of the tumors was then monitored. At the beginning of administration, 7 mice having tumors in similar size were assigned to each group. Here, the administration group was administered intratumorally (i.t, 6 μg/head) or intravenously (i.v, 30 μg/head). During the experimental period, the body weight of the mice and the tumor size were measured 2 to 3 times a week, and the tumor size in each group was monitored until Day 29 starting from the starting day of administration.

When administered to the pancreatic cancer xenograft model mouse, the p53 mRNA anticancer therapeutic agent not only showed significant tumor growth inhibition efficacy in the Mia-PaCa-2 xenograft model compared to the control group (administered with empty LNP as a vehicle) (FIG. 3A), but also appeared to be safe in terms of side effects with no decrease in the body weight of the mouse after 4 weeks of continuous mRNA administration (FIG. 3B). On Day 21 after administration, the tumor inhibition ability was calculated to 63.0 % and 50.2 % in the i.t administration group and the i.v administration group, respectively, confirming that the anticancer efficacy was exhibited regardless the route of administration.

**Example 8: Evaluation of anticancer efficacy of p53 mRNA anticancer candidate agent in lung cancer xenograft model**

To evaluate the anticancer efficacy of the p53 mRNA according to the present disclosure, the p53 mRNA was administered to a lung cancer mouse model in which the H1299 cells were xenografted, and changes in tumor size were observed. To the administration group, a p53 mRNA anticancer therapeutic agent was administered three times a week (6 times in total, 45 μg/head) for 3 weeks.

Specifically, the H1299 cells (ATCC) were injected subcutaneously into the dorsal side of a 5-week-old BALB/c nude mouse. When tumors were observed with the naked eye a few days later, the size of the tumors was then monitored. At the beginning of administration, 6 mice having tumors in similar size were assigned to each group. Here, the administration group was administered intratumorally (i.t, 6 μg/head) or intravenously (i.v, 45 μg/head). During the experimental period, the body weight of the mice and the tumor size were measured 2 to 3 times a week, and the tumor size in each group was monitored until Day 15 starting from the starting day of administration.

When administered to the lung cancer xenograft model mouse, the p53 mRNA anticancer therapeutic agent not only showed significant tumor growth inhibition efficacy in the H1299 xenograft model compared to the control group (administered with empty LNP as a vehicle) (FIG. 4A), but also appeared to be safe in terms of side effects with no decrease in the body weight of the mouse after 2 weeks of continuous mRNA administration (FIG. 4B). On Day 13 after administration, the tumor inhibition ability was calculated to 74.8 % and 32.8 % in the i.t administration group and the i.v administration group, respectively, confirming that i.t administration group exhibited a high cancer growth inhibition

effect.

## Claims

1. An mRNA comprising a non-natural 5' untranslated region (5'UTR), a sequence encoding a polypeptide, and a non-natural 3' untranslated region (3'UTR),

    wherein the 5'UTR does not form a secondary structure,
    the sequence encoding a polypeptide is a sequence encoding p53 or a functional fragment thereof, and
    the 3'UTR has: a secondary structure represented by dot-bracket notation below:

    .

    (((((...........(((...(((((.(((....))).)))))...)))..(((((((..((((....((((((.(((((.......)))))..))-
    ).)))) )))...))))))).......(((....)))............(((.........(((....))))))..)))); or
    a secondary structure represented by dot-bracket notation below:
    ((((((((((((...(((((.((........((((((((.........)))))))))))))))).....(((...))).....))))))))))))..(-
    (((((( (((((....))))))...)))))).....((((((...))))))..........
    wherein, in the dot-bracket notation, a dot denotes unpaired bases in a sequence, and "(" and ")" denote paired bases in a sequence.

2. The mRNA of claim 1, wherein the mRNA comprising the non-natural 5'UTR has a sequence of Formula 1:

    Formula 1: $AGN_aGCCACC$

    wherein N is A, U, G, or C, each N is identical to or different from each other, and a indicates the number of repeats of N and is 42 or 62.

3. The mRNA of claim 1, wherein the mRNA comprising the 3'UTR has a mean free energy of -0.1 to -0.3 kcal/mol for RNA folding,

    (a) the secondary structure of the mRNA comprising the 3'UTR, represented by .(((((...........(((...(((((.(((....))).)))))...)))..(((((((..((((....((((((.(((((.......)))))..))).))))-
    )))...))))))).......(((....)))............(((.........(((....))))))..))))), has a length of 178 nt and a GC-content (GC%) of 40 to 50 %, and
    (b) the secondary structure of the mRNA comprising the 3'UTR, represented by
    ((((((((((((...(((((.((........((((((((.........)))))))))))))))).....(((...))).....))))))))))))..(-
    (((((( (((((....))))))...)))))).....((((((...))))))..........., has a length of 158 nt and a GC% of 30 to 50 %.

4. The mRNA of claim 1, wherein the 5'UTR comprises a nucleotide sequence of any one of SEQ ID NOs: 1 to 9, and the 3'UTR comprises a nucleotide sequence of any one of SEQ ID NOs: 10 to 17.

5. The mRNA of claim 4, wherein the mRNA further comprises a Kozak sequence or a poly (A) sequence.

6. The mRNA of claim 1, wherein the mRNA has one or more modified nucleotides.

7. The mRNA of claim 1, wherein the mRNA comprises a 5'-cap selected from Cap0, Cap1, and Cap2.

8. DNA encoding the mRNA of any one of claims 1 to 7.

9. A composition comprising, as an active ingredient, the mRNA of any one of claims 1 to 7 or DNA encoding the mRNA.

10. The composition of claim 9, wherein the composition is a pharmaceutical composition for preventing or treating cancer.

11. The composition of claim 10, wherein the cancer is caused by a p53 mutation.

12. A method of preventing or treating cancer in a subject, the method comprising administering a therapeutically effective amount of the mRNA of any one of claims 1 to 7 to a subject.

FIG. 1

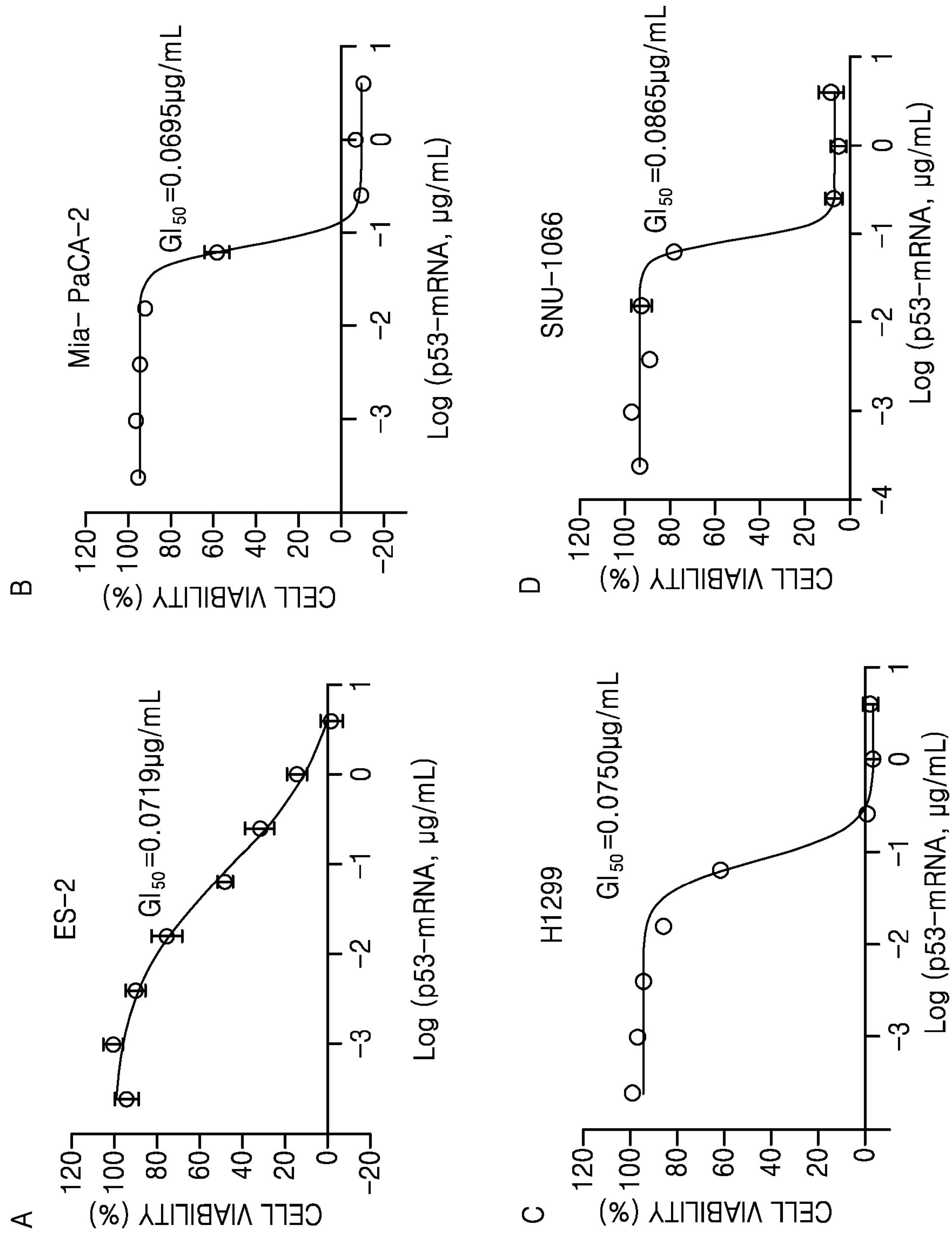
A
ES-2
$GI_{50}$=0.0719μg/mL
CELL VIABILITY (%)
Log (p53-mRNA, μg/mL)
B
Mia- PaCA-2
$GI_{50}$=0.0695μg/mL
CELL VIABILITY (%)
Log (p53-mRNA, μg/mL)
C
H1299
$GI_{50}$=0.0750μg/mL
CELL VIABILITY (%)
Log (p53-mRNA, μg/mL)
D
SNU-1066
$GI_{50}$=0.0865μg/mL
CELL VIABILITY (%)
Log (p53-mRNA, μg/mL)

FIG. 2

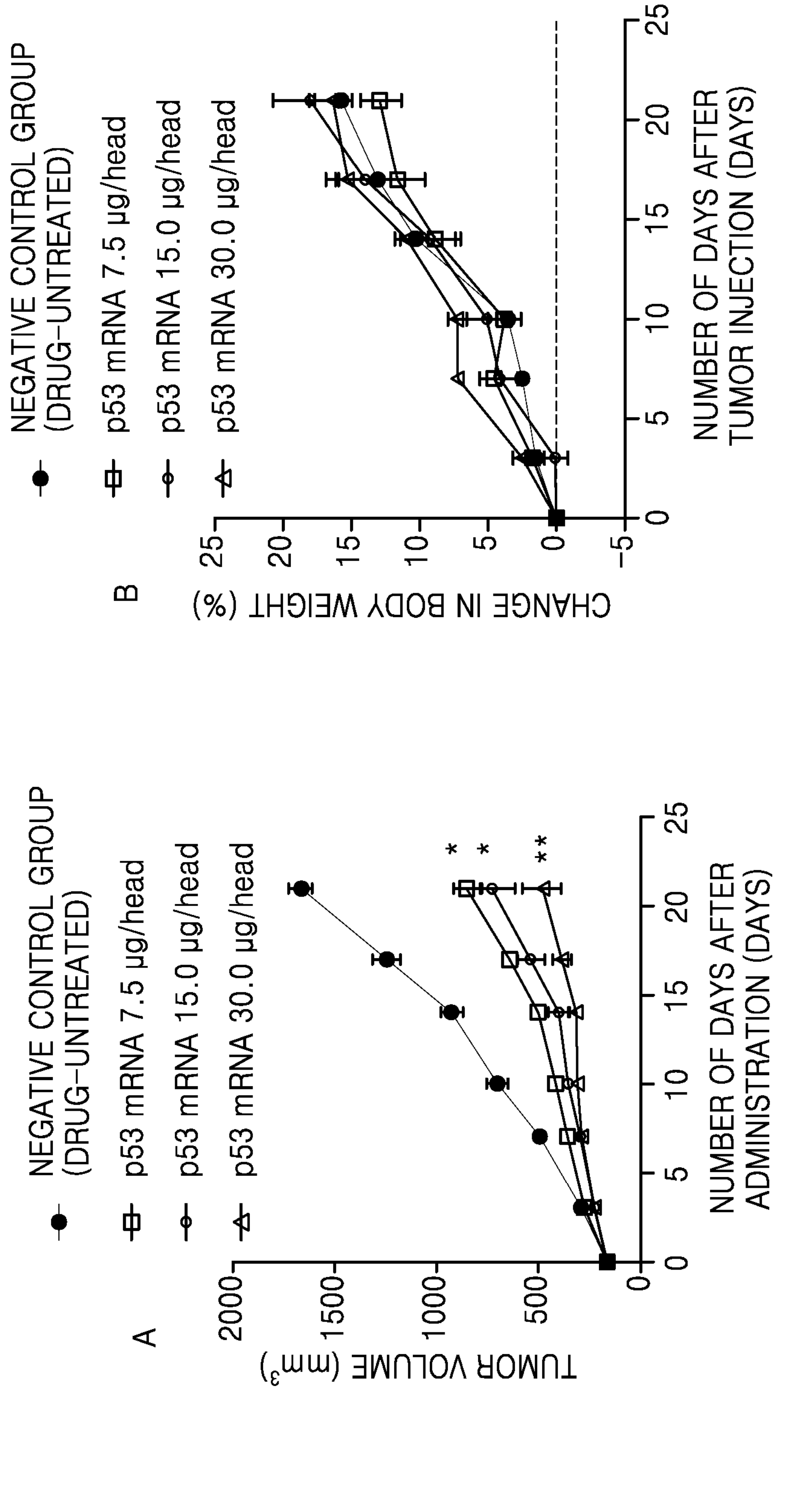
A
NEGATIVE CONTROL GROUP (DRUG-UNTREATED)
p53 mRNA 7.5 μg/head
p53 mRNA 15.0 μg/head
p53 mRNA 30.0 μg/head
TUMOR VOLUME (mm³)
2000
1500
1000
500
0
0
5
10
15
20
25
NUMBER OF DAYS AFTER ADMINISTRATION (DAYS)
*
*
**
B
NEGATIVE CONTROL GROUP (DRUG-UNTREATED)
p53 mRNA 7.5 μg/head
p53 mRNA 15.0 μg/head
p53 mRNA 30.0 μg/head
CHANGE IN BODY WEIGHT (%)
25
20
15
10
5
0
-5
0
5
10
15
20
25
NUMBER OF DAYS AFTER TUMOR INJECTION (DAYS)

FIG. 3

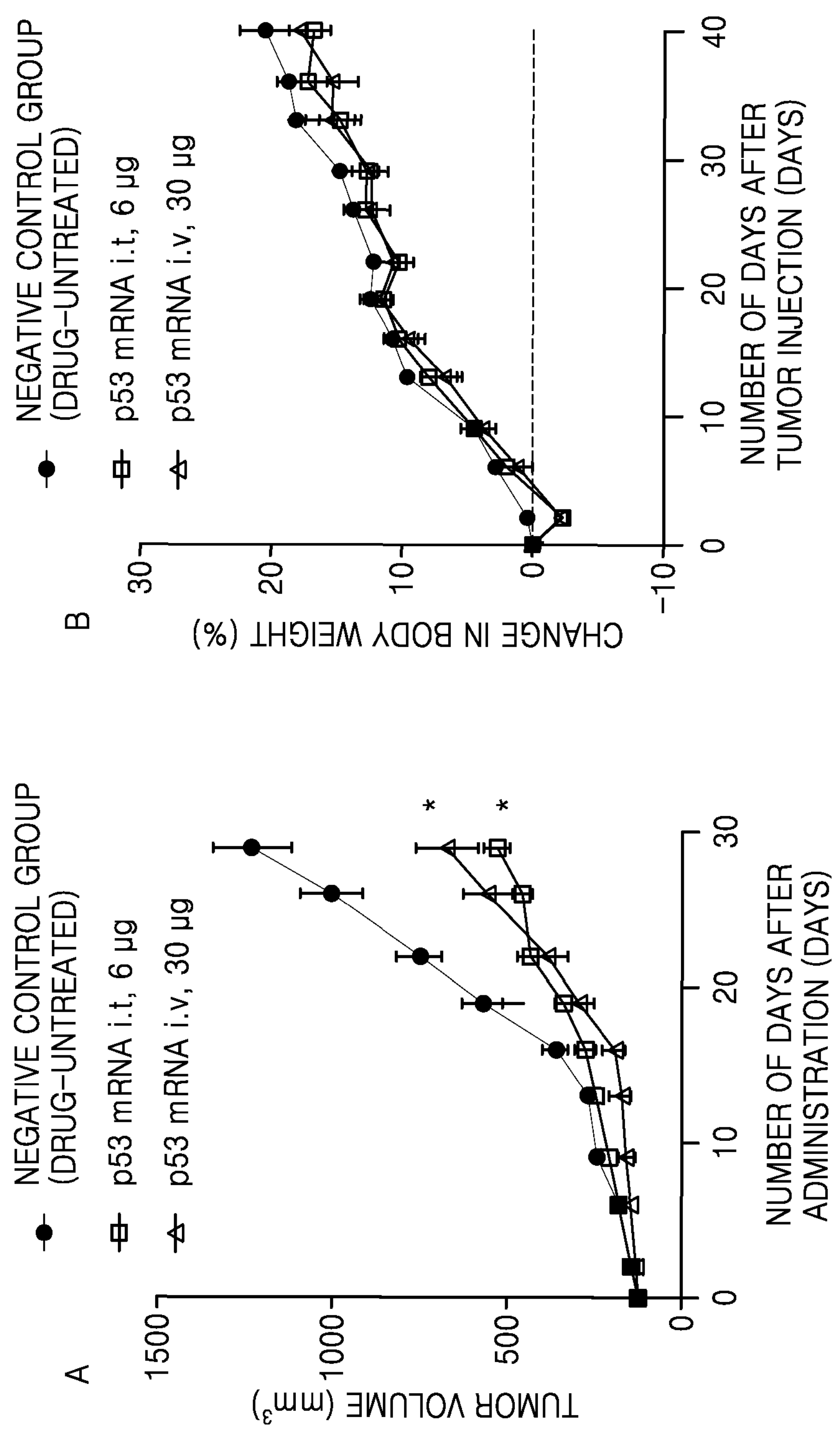
A
TUMOR VOLUME ($mm^3$)
1500
1000
500
0
0
10
20
30
NUMBER OF DAYS AFTER ADMINISTRATION (DAYS)
NEGATIVE CONTROL GROUP (DRUG-UNTREATED)
p53 mRNA i.t, 6 μg
p53 mRNA i.v, 30 μg
*
*
B
CHANGE IN BODY WEIGHT (%)
30
20
10
0
-10
0
10
20
30
40
NUMBER OF DAYS AFTER TUMOR INJECTION (DAYS)
NEGATIVE CONTROL GROUP (DRUG-UNTREATED)
p53 mRNA i.t, 6 μg
p53 mRNA i.v, 30 μg

FIG. 4

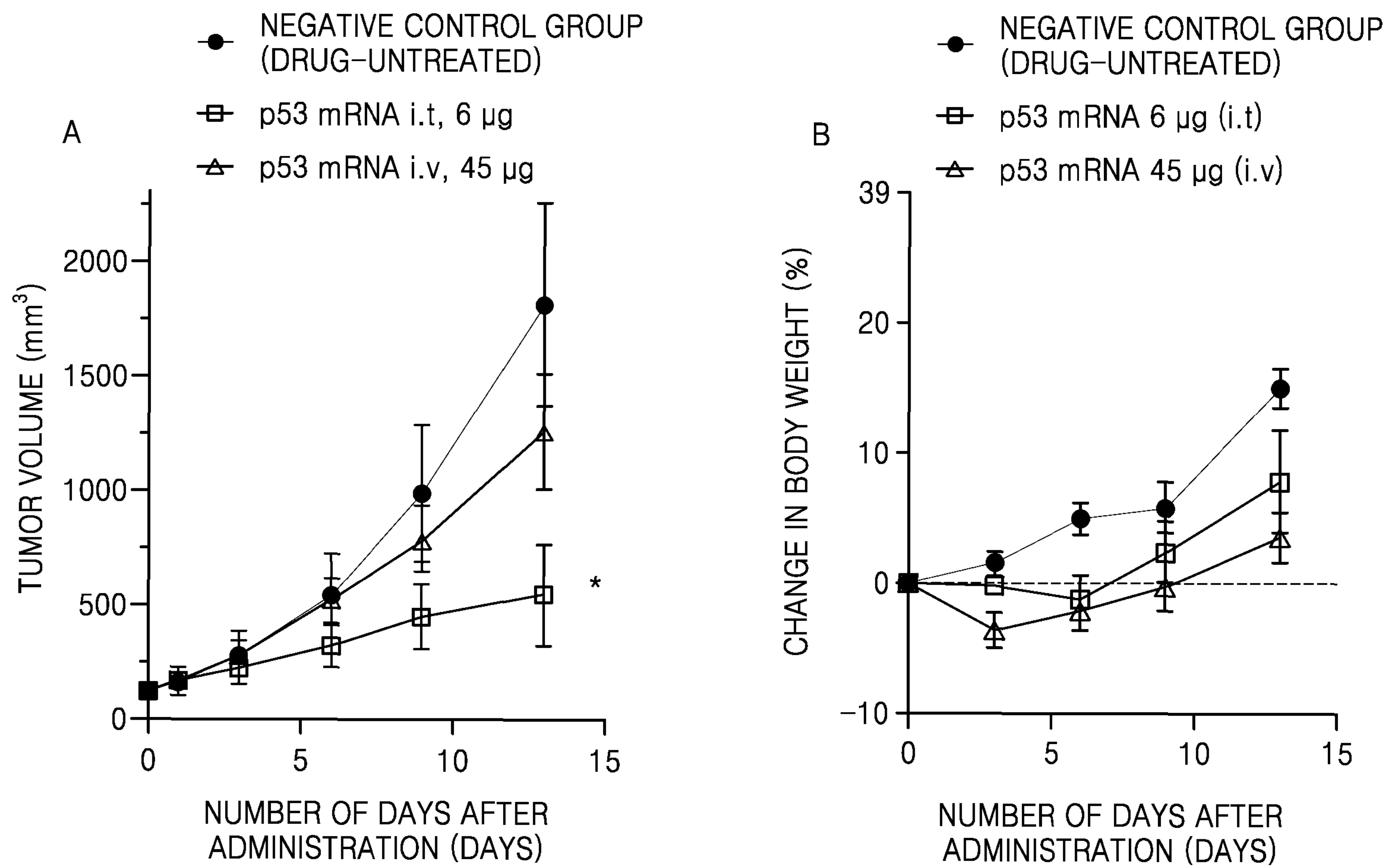
A
NEGATIVE CONTROL GROUP (DRUG-UNTREATED)
p53 mRNA i.t, 6 μg
p53 mRNA i.v, 45 μg
TUMOR VOLUME (mm³)
2000
1500
1000
500
0
0
5
10
15
*
NUMBER OF DAYS AFTER ADMINISTRATION (DAYS)
B
NEGATIVE CONTROL GROUP (DRUG-UNTREATED)
p53 mRNA 6 μg (i.t)
p53 mRNA 45 μg (i.v)
CHANGE IN BODY WEIGHT (%)
39
20
10
0
−10
0
5
10
15
NUMBER OF DAYS AFTER ADMINISTRATION (DAYS)

## INTERNATIONAL SEARCH REPORT

International application No.
**PCT/KR2024/014752**

**A. CLASSIFICATION OF SUBJECT MATTER**

**A61K 48/00**(2006.01)i; **A61K 38/17**(2006.01)i; **A61P 35/00**(2006.01)i; **C12N 15/67**(2006.01)i; **C07K 14/47**(2006.01)i; **C12N 15/11**(2006.01)i; **C12N 15/113**(2010.01)i; **C12N 15/63**(2006.01)i

According to International Patent Classification (IPC) or to both national classification and IPC

**B. FIELDS SEARCHED**

Minimum documentation searched (classification system followed by classification symbols)

A61K 48/00(2006.01); A01K 67/027(2006.01); A61K 39/00(2006.01); C12N 15/113(2010.01); C12N 15/63(2006.01); C12N 15/67(2006.01); C12N 15/79(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 5'-UTR, 3'-UTR, 비천연(unnatural), mRNA, p53

**C. DOCUMENTS CONSIDERED TO BE RELEVANT**

| Category* | Citation of document, with indication, where appropriate, of the relevant passages | Relevant to claim No. |
|---|---|---|
| A | KR 10-2023-0083893 A (KOREA UNIVERSITY RESEARCH AND BUSINESS FOUNDATION et al.) 12 June 2023 (2023-06-12)<br>See claims 1, 4, 8, 12 and 13; and paragraph [0158]. | 1-11 |
| A | KR 10-2023-0000471 A (HANMI PHARM. CO., LTD.) 02 January 2023 (2023-01-02)<br>See claims 1 and 3; and paragraph [0111]. | 1-11 |
| A | KR 10-2023-0096863 A (HANMI PHARM. CO., LTD.) 30 June 2023 (2023-06-30)<br>See paragraphs [0021], [0089] and [0111]. | 1-11 |
| A | KR 10-1541935 B1 (INTREXON CORPORATION) 05 August 2015 (2015-08-05)<br>See paragraph [0045]. | 1-11 |
| A | KR 10-2312903 B1 (ETHRIS GMBH) 15 October 2021 (2021-10-15)<br>See abstract; and claim 1. | 1-11 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 January 2025** | **09 January 2025** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

**INTERNATIONAL SEARCH REPORT**

International application No.
**PCT/KR2024/014752**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/KR2024/014752**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **12**
because they relate to subject matter not required to be searched by this Authority, namely:
Claim 12 pertains to a method for treatment of the human body [PCT Article 17(2)(a)(i) and PCT Rule 39.1(iv)].

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/KR2024/014752**

| Patent document cited in search report | Publication date (day/month/year) | Patent family member(s) | Publication date (day/month/year) |
|---|---|---|---|
| KR 10-2023-0083893 A | 12 June 2023 | AU 2022-401495 A1 | 08 June 2023 |
| | | CA 3239776 A1 | 08 June 2023 |
| | | CN 118339293 A | 12 July 2024 |
| | | EP 4441222 A1 | 09 October 2024 |
| | | TW 202323527 A | 16 June 2023 |
| | | WO 2023-101508 A1 | 08 June 2023 |
| KR 10-2023-0000471 A | 02 January 2023 | AU 2022-297171 A1 | 29 December 2022 |
| | | CA 3223855 A1 | 29 December 2022 |
| | | CN 117597446 A | 23 February 2024 |
| | | EP 4361270 A1 | 01 May 2024 |
| | | JP 2024-528447 A | 30 July 2024 |
| | | TW 202317766 A | 01 May 2023 |
| | | US 2024-0301433 A1 | 12 September 2024 |
| | | WO 2022-270969 A1 | 29 December 2022 |
| KR 10-2023-0096863 A | 30 June 2023 | CN 118434871 A | 02 August 2024 |
| | | EP 4455291 A1 | 30 October 2024 |
| | | WO 2023-121131 A1 | 29 June 2023 |
| KR 10-1541935 B1 | 05 August 2015 | AU 2008-304201 A1 | 02 April 2009 |
| | | AU 2008-304201 B2 | 14 August 2014 |
| | | AU 2008-304201 C1 | 05 February 2015 |
| | | CA 2715078 A1 | 02 April 2009 |
| | | CA 2715078 C | 23 July 2019 |
| | | CN 101855233 A | 06 October 2010 |
| | | CN 101855233 B | 07 May 2014 |
| | | EP 2205618 A2 | 14 July 2010 |
| | | EP 2205618 B1 | 09 November 2016 |
| | | EP 2535419 A2 | 19 December 2012 |
| | | EP 2535419 A3 | 29 May 2013 |
| | | EP 3156414 A1 | 19 April 2017 |
| | | EP 3156414 B1 | 04 December 2019 |
| | | JP 2010-539946 A | 24 December 2010 |
| | | JP 5514727 B2 | 04 June 2014 |
| | | KR 10-1541935 B1 | 05 August 2015 |
| | | US 2010-0293625 A1 | 18 November 2010 |
| | | US 8835621 B2 | 16 September 2014 |
| | | WO 2009-042971 A2 | 02 April 2009 |
| | | WO 2009-042971 A3 | 14 May 2009 |
| | | WO 2009-042971 A8 | 16 July 2009 |
| KR 10-2312903 B1 | 15 October 2021 | AU 2017-242794 A1 | 05 October 2017 |
| | | AU 2017-242794 B2 | 15 December 2022 |
| | | AU 2023-201593 A1 | 27 April 2023 |
| | | CA 3018904 A1 | 05 October 2017 |
| | | CA 3018904 C | 02 April 2024 |
| | | CN 109154001 A | 04 January 2019 |
| | | CN 109154001 B | 18 July 2023 |
| | | CN 116814614 A | 29 September 2023 |
| | | EP 3436589 A1 | 06 February 2019 |
| | | EP 3436589 B1 | 23 September 2020 |
| | | JP 2019-512260 A | 16 May 2019 |
| | | JP 2021-184745 A | 09 December 2021 |

Form PCT/ISA/210 (patent family annex) (July 2022)

**INTERNATIONAL SEARCH REPORT**
**Information on patent family members**

International application No.
**PCT/KR2024/014752**

| Patent document cited in search report | Publication date (day/month/year) | Patent family member(s) | Publication date (day/month/year) |
|---|---|---|---|
| | | JP 2023-134529 A | 27 September 2023 |
| | | JP 6959654 B2 | 02 November 2021 |
| | | KR 10-2312903 B1 | 15 October 2021 |
| | | US 11352638 B2 | 07 June 2022 |
| | | US 11981910 B2 | 14 May 2024 |
| | | US 2019-0144883 A1 | 16 May 2019 |
| | | US 2023-0086606 A1 | 23 March 2023 |
| | | WO 2017-167910 A1 | 05 October 2017 |

Form PCT/ISA/210 (patent family annex) (July 2022)